# EUROPÄISCHE PATENTSCHRIFT

(11) **EP 3 362 788 B1**
(45) Veröffentlichungstag und Bekanntmachung des Hinweises auf die Patenterteilung: **11.12.2024**
(21) Anmeldenummer: 16781742.8
(22) Anmeldetag: 11.10.2016
(51) Int. Cl.: G01N 30/16, G01N 30/32, G01N 30/38, G01N 33/00, G01N 30/00, G01N 30/02, G01N 30/88

(54) **KONTINUIERLICHE UND TRENNENDE GASANALYSE**
CONTINUOUS AND SEPARATING GAS ANALYSIS
ANALYSE DE GAZ EN CONTINU ET PAR SÉPARATION

(30) Priorität: 13.10.2015 DE 102015219838
(43) Veröffentlichungstag der Anmeldung: 22.08.2018
(73) Patentinhaber: Dräger Safety AG & Co. KGaA, 23560 Lübeck (DE)
(72) Erfinder: SCHMITTMANN, Matthias, 20146 Hamburg (DE); WEBER, Johannes, 20099 Hamburg (DE); JÜNEMANN, Arne, 21075 Hamburg (DE); WEBER, Paul, 13357 Berlin (DE)
(74) Vertreter: Meyer-Gramann, Klaus Dieter
(86) Internationale Anmeldenummer: PCT/EP2016/074315
(87) Internationale Veröffentlichungsnummer: WO 2017/064047

(56) Entgegenhaltungen:
- US-A- 6 165 251
- US-A1- 2005 284 209
- US-A1- 2009 101 017
- US-A1- 2011 005 300
- US-A1- 2011 259 081
- STEFANIE SIELEMANN: "Detektion flüchtiger organischer Verbindungen mittels Ionenmobilitätsspektrometrie und deren Kopplung mit Multi-Kapillar-Gas-Chromatographie", 1 November 1999 (1999-11-01), XP055329040, Retrieved from the Internet <URL:https://eldorado.tu-dortmund.de/bitstream/2003/2411/2/sielemansig.pdf> [retrieved on 20161214]

## Beschreibung

Die Erfindung betrifft eine tragbare Gasanalysevorrichtung zum Durchleiten eines Gasflusses, insbesondere für leicht flüchtige Verbindungen.

Leicht flüchtige organische Substanzen sind häufig gesundheitsschädlich. In der Umwelt und um Betriebsanlagen müssen aus diesem Grund gesetzliche Höchstwerte für deren Konzentration eingehalten werden. Die Messung der Konzentration der leicht flüchtigen Verbindungen kann mit mobilen Messgeräten durchgeführt werden.

Die Messung zur Erfassung der Einzel-Konzentrationen der leicht flüchtigen Verbindungen in einem Probengasgemisch erfordert einen relativ hohen Zeitaufwand von mindestens mehreren Minuten. Für eine ortsaufgelöste Messung der Einzelkonzentrationen in großen Gebieten oder großen Gebäuden wird daher sehr viel Zeit benötigt. Es wird aus diesem Grund zunächst eine schnelle Integrationsmessung (ca. 2 Sekunden) durchgeführt, um zu erfassen, an welchen Orten leicht flüchtige Verbindungen in höherer Konzentration vorhanden sind. Es ist intern bekannt, dass bei mobilen Messgeräten ein Gasstrom aus dem Probengasgemisch auf einen Detektor geführt wird, der die Gesamt-Konzentration der leicht flüchtigen Verbindungen im Probengas erfasst. Wenn leicht flüchtige Verbindungen gefunden werden, wird der Gasstrom des Probengasgemischs mittels Ventile auf einen anderen Detektor geführt, der die Einzel-Konzentrationen der leicht flüchtigen Verbindungen im Probengas misst. Solche Messgeräte sind beispielsweise bekannt aus den Dokumenten US 2011/0259081 A1, US 2005/0284209 A1 oder US 6,165,251 A.

Diese bekannten Messgeräte haben den Nachteil, dass für die Dauer der Messung der Einzel-Konzentrationen die Suche nach weiteren Orten, an denen höhere Konzentrationen der leicht flüchtigen Verbindungen vorhanden sein könnten, unterbrochen werden muss.

Herkömmliche Messgeräte, die eine gleichzeitige Messung erlauben, weisen für jeden Messmodus einen separaten Messweg mit separaten Öffnungen, Gebläsen, Ventilen und Leitungen für das Probengas auf. Durch diese Mehrfachstruktur werden diese Messgeräte schwer und wartungsintensiv. Ein Messgerät mit ungünstiger Mehrfachstruktur ist beispielsweise bekannt aus dem Dokument US 2011/0259081 A1).

Der Erfindung liegt die Aufgabe zu Grunde, eine hinsichtlich Kompaktheit und Schnelligkeit verbesserte Gasanalysevorrichtung zu schaffen, die verbrauchs- und verschleißgünstig ist. Die Aufgabe wird gelöst durch die Merkmale der unabhängigen Ansprüche. Vorteilhafte Weiterbildungen sind Gegenstand der abhängigen Ansprüche.

Bei einer Gasanalysevorrichtung zum Durchleiten eines Gasflusses, insbesondere für leicht flüchtige Verbindungen, wobei die Gasanalysevorrichtung diu, einen Suchermesspfad und einen Trennmesspfad umfasst, wobei der Suchermesspfad sich von einer Probengaseintrittsöffnung zu einer ersten Abluftöffnung erstreckt, wobei ein Verbindungspfad von dem Suchermesspfad abzweigt zu dem Trennmesspfad, wobei der Trennmesspfad sich von dem Verbindungspfad zu einer zweiten Abluftöffnung erstreckt und mit einer Trägergaseintrittsöffnung verbunden ist, und wobei am Trennmesspfad ein Trenn-Detektor angeordnet ist, ist erfindungsgemäß vorgesehen, dass die Gasanalysevorrichtung tragbar ist und ein Steuerelement aufweist, das zum Umkehren des Gasflusses im Verbindungspfad derart ausgebildet ist, dass bei umgekehrter Richtung ein Teil von einem an der Probeneintrittsöffnung eintretenden Probengasgemisch über den Verbindungspfad in den Trennmesspfad fließt und der andere Teil dem Suchermesspfad folgt, wobei im Suchermesspfad ein Sucher-Detektor angeordnet ist, der zum kontinuierlichen Erkennen der Anwesenheit einer Gruppe von leicht flüchtigen Verbindungen ausgebildet ist.

Zunächst seien einige Begriffe näher erläutert:
Unter einem Suchermesspfad wird der Weg des Probengasgemisches in der Gasanalysevorrichtung verstanden, wobei auf dem Weg die Anwesenheit von leicht flüchtigen Verbindungen erfasst wird. Der Suchermesspfad dient dazu, die leicht flüchtigen Verbindungen zu suchen.

Unter einem Trennmesspfad wird der Weg des Probengasgemisches in der Gasanalysevorrichtung verstanden, wobei auf dem Weg die Komponenten des Gemisches getrennt werden, um die Einzel-Konzentrationen zu erfassen. Der Trennmesspfad dient damit dazu, ein Gasgemisch in seine Bestandteile zu trennen, um die Konzentrationen der einzelnen Verbindungen zu erfassen.

Die Erfindung basiert auf dem Gedanken, die Messung der Einzel-Konzentrationen der leicht flüchtigen Verbindungen durch die Richtung des Gasflusses im Verbindungspfad zu steuern. Solange keine Messung durchgeführt wird, fließt Trägergas von dem Verbindungspfad in den Suchermesspfad. Indem die Richtung des Gasflusses im Verbindungspfad durch das Steuerelement umgekehrt wird, fließt ein Teil des Probengasgemisches von dem Suchermesspfad über den abzweigenden Verbindungspfad in den Trennmesspfad, so dass die Einzel-Konzentrationen gemessen werden können. Dabei verbleibt der andere Teil des Probengases im Suchermesspfad, so dass dank dieser Aufteilung des Probegases und des dort angeordneten Sucherdetektors die Suche nach der Anwesenheit von leicht flüchtigen Verbindungen weitergeführt werden kann. Der Gasfluss kann durch Flusserzeugerelementen wie z. B. Gebläseeinheiten und/oder anderen geeigneten Vorrichtungen wie Flussrichtern und Ventilen erzeugt werden.

Dabei wird zwischen dem Verbindungsmesspfad und dem Suchermesspfad kein Ventil benötigt. Die Umkehr des Gasflusses wird vielmehr durch ein Steuerelement bewirkt, das die Druckverhältnisse zwischen dem Trennmesspfad und dem Suchermesspfad steuert. Es kann so bei umgekehrter Richtung ein Teil von dem an der Probeneintrittsöffnung eintretenden Probengasgemisch über den Verbindungspfad in den Trennmesspfad fließen und der andere Teil dem Suchermesspfad folgen. Die Steuerung der Richtung des Gasflusses kann durch eine Änderung des Druckunterschieds zwischen dem Suchermesspfad und dem Trennmesspfad bewirkt werden.

Bei der Suche, ob überhaupt leicht flüchtige Verbindungen an den Messorten vorhanden sind, bewirkt das Steuerelement, dass der Druck im Trennmesspfad höher als im Suchermesspfad ist. Dadurch strömt Trägergas von der Trägergaseintrittsöffnung durch den Verbindungspfad in den Suchermesspfad. Das durch die Probengaseintrittsöffnung strömende Probengas wird dadurch daran gehindert, in den Trennmesspfad einzuströmen. Im Suchermesspfad kann das Probengas auf leicht flüchtige Verbindungen analysiert werden. Dazu ist im Suchermesspfad ein Sucher-Detektor angeordnet, der zum kontinuierlichen Erkennen der Anwesenheit einer Gruppe von leicht flüchtigen Verbindungen ausgebildet ist. Das Probengas und das Trägergas entweichen in diesem Fall durch die erste Abluftöffnung.

Wenn leicht flüchtige Verbindungen im Suchermesspfad erfasst wurden, kann das Steuerelement, vorzugsweise automatisch, die Druckverhältnisse in der Gasanalysevorrichtung so einstellen, dass im Trennmesspfad ein niedrigerer Druck als im Suchermesspfad herrscht. Dadurch wird ein Teil des Probengases von dem Suchermesspfad durch den abzweigenden Verbindungspfad in den Trennmesspfad geleitet. Im Trennmesspfad können die Konzentrationen der einzelnen Verbindungen im Probengas erfasst werden. Das Probengas entweicht in diesem Fall durch die zweite Abluftöffnung.

Da lediglich ein Teil des durch den Suchermesspfad strömenden Probengases durch den Verbindungspfad strömt, kann die Suche nach leicht flüchtigen Verbindungen weitergeführt werden, während die Einzel-Konzentrationen der leicht flüchtigen Verbindungen erfasst werden. Dies beschleunigt die Vermessung von großen Gebieten bzw. großen Gebäuden. Die Erfindung nutzt weiter die Messpfade effizient und vermeidet auf diese Weise eine Mehrfachstruktur. Dies verringert das Gewicht und den Verschleiß der Komponenten der Gasanalysevorrichtung. Weiter wird durch die Vermeidung eines Ventils zwischen dem Verbindungspfad und dem Suchermesspfad die Wartung der tragbaren Gasanalysevorrichtung vereinfacht und die Lebensdauer der Vorrichtung erhöht, da die Anzahl der mechanischen Bauteile klein gehalten wird.

Vorteilhafterweise ist der Sucher-Detektor ein Photoionisationsdetektor, ein Halbleiter-Gasdetektor oder ein Wärmeleitfähigkeitsdetektor.

Das Steuerelement kann vorteilhafterweise zur automatischen Umkehr des Gasflusses im Verbindungspfad, wenn die Anwesenheit von leicht flüchtigen Verbindungen detektiert wird, ausgebildet sein.

Weiter ist mit Vorteil der Trenn-Detektor zur Mengenbestimmung von leicht flüchtigen Verbindungen ausgebildet ist und vorzugsweise ein Gaschromatograph in Kombination mit einem Photoionisationsdetektor, mit einem Wärmeleitfähigkeitsdetektor, mit einem Halbleiter-Gasdetektor oder mit einem Massenspektrometer ist. Dazu ist es zweckmäßig, dass der Trenn-Detektor eine Trennsäule umfasst, wobei die Trennsäule als eine Multi-Kapillar-Säule ausgebildet ist. Die Trennsäule fungiert dabei als Retentionselement, durch das die einzelnen leicht flüchtigen Verbindungen mit unterschiedlichen Geschwindigkeiten strömen. Die Multi-Kapillare führt zu höherem Gasvolumenstrom (stärkeren Signalen) und folglich niedrigeren Nachweisgrenzen. In Verbindung mit der Kürze der Säule (und ihre Materialbeschaffenheit) führt dies zu den schnellen Analysen. Somit erfolgt die Bestimmung der Konzentration einer leicht flüchtigen Verbindung sehr schnell und kann in weniger als 30 Sekunden durchgeführt werden. Dies ist vor allem für tragbare Gasanalysevorrichtungen ein großer Vorteil, um direkt vor Ort schnell Messergebnisse erhalten zu können.

Bevorzugt sind die erste Abluftöffnung und die zweite Abluftöffnung innerhalb eines Gehäuses kombiniert zu einer gemeinsamen Abluftöffnung. Somit nutzen der Trennmesspfad und der Sucherpfad eine gemeinsame Abluftöffnung. Damit kann die Gasanalysevorrichtung noch kompakter ausgeführt sein, was insbesondere bei portabler Nutzung einen beträchtlichen Anwendungsvorteil für sich buchen kann.

Zweckmäßigerweise ist ein gesamter - von dem Suchermesspfad, dem Trennmesspfad bis zum Trenn-Detektor und dem Verbindungspfad gebildeter - analytischer Pfad frei von Ventilen ausgeführt, d. h. ventillos. Mit dem Verzicht auf Ventile in diesem Bereich wird der Gefahr von analyseverfälschenden Anreicherungen im Ventilbereich auf wirksame Weise begegnet. Im Übrigen kann so die Messgeschwindigkeit erhöht und der Verschleiß verringert werden.

Vom Verbindungsmesspfad zweigt vorteilhafterweise ein Filterpfad ab, der mit der Trägergaseintrittsöffnung verbunden ist, wobei in dem Filterpfad ein Filterelement angeordnet ist. Das Filterelement wird dazu genutzt, um aus dem durch die Trägergaseintrittsöffnung strömenden Trägergas leicht flüchtige Verbindungen zu filtern. Das gefilterte Trägergas kann danach als Spülgas im Trennmesspfad eingesetzt werden, um zwischen zwei Messungen der Einzelkonzentrationen Reste des Probengases aus der letzten Messung auszuspülen. Wenn im Trennmesspfad ein höherer Druck als im Sucherpfad herrscht, kann das Trägergas von der Trägergaseintrittsöffnung zuerst durch das Filterelement strömen. Danach strömt das Trägergas durch den Trennmesspfad. Gleichzeitig kann das Trägergas durch den Verbindungspfad in den Suchermesspfad fließen. Der Filterpfad kann als Bypass zum Trenn-Detektor ausgebildet sein. Wenn der Druck im Trennmesspfad niedriger als im Suchermesspfad ist, gelangt kein Trägergas von dem Filterpfad in den Verbindungspfad.

Zweckmäßigerweise ist das Steuerelement dazu in einer ersten vorteilhaften Ausführungsform als ein Ventil ausgebildet, das zum Öffnen und Sperren des Filterpfads ausgebildet ist. In diesem Fall wird das Trägergas nach dem Sperren des Filterpfads direkt von der Trägergaseintrittsöffnung durch die zweite Abluftöffnung geleitet. In einer anderen Ausführungsform kann der Trägergasfluss an der Trägergaseintrittsöffnung blockiert werden, so dass das Trägergas gar nicht erst in die Gasanalysevorrichtung bzw. den Filterpfad strömt.

In einer zweiten vorteilhaften Ausführungsform ist das Steuerelement als eine regelbare Gebläseeinheit an der Trägergaseintrittsöffnung, an der ersten Abluftöffnung und/oder an dem Trenn-Detektor ausgebildet. Durch das Auf- oder Abregeln der Gebläseleistung wird der Druck im Suchermesspfad bzw. im Trennmesspfad beeinflusst. Mit der regelbaren Gebläseeinheit kann damit der Druck im Trennmesspfad im Vergleich zum Suchermesspfad eingestellt werden. Dabei kann entweder der Druck im Suchermesspfad oder im Trennmesspfad verändert werden. Wenn sowohl im Suchermesspfad als auch im Trennmesspfad regelbare Gebläseeinheiten angeordnet sind, können die Drücke im Suchermesspfad und im Trennmesspfad derart gegensätzlich verändert werden, dass die Gasflussrichtung im Verbindungspfad umgekehrt wird. Es kann aber auch vorgesehen sein, dass an der ersten Abluftöffnung eine erste regelbare Gebläseeinheit als Flusserzeugerelement angeordnet ist, wobei die erste regelbare Gebläseeinheit einen Gasfluss in Richtung der ersten Abluftöffnung erzeugt, und zwischen dem Trenndetektor und der zweiten Abluftöffnung eine zweite Gebläseeinheit angeordnet ist, die einen Gasfluss vom Verbindungspfad durch den Trenndetektor in Richtung der zweiten Abluftöffnung erzeugt. Es wird so mittels der Gebläseeinheiten die Richtung des Gasflusses im Verbindungspfad bestimmt. Dazu ist es weiter zweckmäßig, wenn an der Trägergaseintrittsöffnung ein Durchfluss- oder Druckdifferenzsensor angeordnet ist, wobei die Leistung der Gebläseeinheit am Trenn-Detektor in Abhängigkeit der Messung des Durchfluss- oder Druckdifferenzsensors geregelt wird. Damit kann die Regelung der Gebläseeinheiten auf Basis des Trägergasdurchflusses geregelt werden. In beiden Ausführungsformen wird die Steuerung der Gasflüsse in der Gasanalysevorrichtung vereinfacht, da auf komplexe Ventil- und Gebläsesteuerungen verzichtet werden kann.

Weitere Vorteile des Suchers mit Sucher-Detektor sind schnellere Reaktionszeiten und verringerter Verschleiß durch Verunreinigung des Trenn-Detektors. Die Gebläse am Gerät können dank des Suchers kontinuierlich betrieben werden, wodurch sich das Einpendeln der Leistung auf eine kurze Aufwärmphase beschränkt. In einer vorteilhaften Ausführung kann die Leistung danach über einen Regelkreis mit einem Durchflusssensor stabilisiert oder moduliert werden. Durch den Sucher kann die Umkehrung des Gasflusses und damit Injektion für die Trenn-Messung ohne Aufwärmen, Vorspülen oder ähnliche Verfahrensschritte ausgelöst werden. Im Betrieb vor und nach der Injektion reinigt sich das Gerät selbst im Sucherpfad, im Verbindungspfad und nach Beendigung der Trenn-Messung auch im Trennmesspfad, die alle vorteilhaft ohne Ventile ausgeführt sind, an denen es verstärkt zu einer Anreicherung von Stoffen kommt. Durch die so vermiedene Verschmutzung der Gaspfade wird der Verschleiß reduziert und die Lebensdauer der Komponenten erhöht. Der kontinuierliche Betrieb der Gebläse stabilisiert auch die Wärmeentwicklung im Gerät, wodurch es nicht zu einer plötzlichen Erwärmung im Bereich des Trenn-Detektors während der Messung kommt, die etwa im Fall eines Gaschromatographen das Ergebnis stark verzerren würde.

Weiter weist die Gasanalysevorrichtung mit Vorteil ein Ortungsmodul auf, das zum Bestimmen der geographischen Koordinaten der Gasanalysevorrichtung ausgebildet ist und das vorzugsweise mittels einer Signalverbindung mit dem Steuerelement verbunden ist. Die Ortskoordinaten können dabei zusammen mit den Messdaten über die Gesamt- und Einzelkonzentrationen zweckmäßigerweise auf einer Speichereinheit gespeichert werden.

Vorteilhafterweise umfasst die Gasanalysevorrichtung eine interne Auswerteeinheit, die zum Auswerten der Messdaten ausgebildet ist.

Die Erfindung betrifft weiter ein System, das eine tragbare Gasanalysevorrichtung gemäß der vorangegangenen Beschreibung, mit einer internen Auswerteinheit, und eine externe Auswerteeinheit aufweist, wobei erfindungsgemäß vorgesehen ist, dass die interne Auswerteeinheit und die externe Auswerteeinheit zum Austausch von Steuerbefehlen und Messdaten mittels einer drahtlosen Signalverbindung ausgebildet sind. Eine externe Auswerteeinheit kann einfach aufgerüstet werden und verfügt in der Regel über eine höhere Rechenleistung und über fortgeschrittenere Darstellungsmöglichkeiten als gerätinterne Auswertekomponenten.

Weiter betrifft die Erfindung ein Gasanalyseverfahren mittels einer tragbaren Gasanalysevorrichtung nach der vorangegangenen Beschreibung mit den Schritten: Einstellen eines höheren Drucks im Trennmesspfad relativ zum Suchermesspfad, Erkennen der Anwesenheit einer leicht flüchtigen Verbindung aus einer Gruppe von leicht flüchtigen Verbindungen im Suchermesspfad mittels des Sucher-Detektors, Einstellen eines niedrigeren Drucks im Trennmesspfad relativ zum Suchermesspfad, Trennen der einzelnen Mitglieder der Gruppe von leicht flüchtigen Verbindungen, Bestimmen der Menge der getrennten leicht flüchtigen Verbindungen, wobei vorzugsweise das Erkennen der Anwesenheit der leicht flüchtigen Verbindungen während des Trennens und des Bestimmens kontinuierlich weitergeführt wird.

Dabei wird mit Vorteil automatisch ein Anfragesignal auf Grund des Erkennens der Anwesenheit von leicht flüchtigen Verbindungen übermittelt. Vorteilhafterweise wird ein niedrigerer Druck im Trennmesspfad relativ zum Suchermesspfad nach dem Übermitteln eines Anfragesignals eingestellt. Zweckmäßigerweise werden die geographischen Koordinaten des Orts, an dem das Trennen und das Bestimmen der Menge durchgeführt werden, bestimmt und gespeichert.

Die Erfindung wird nachfolgend mit Bezugnahme der beigefügten Zeichnung an Hand von vorteilhaften Ausführungsbeispielen näher erläutert. Es zeigen:
- Fig. 1a,b,c: eine perspektivische Ansicht verschiedener Ausführungsbeispiele für eine tragbaren Gasanalysevorrichtung mit externer Auswerteeinheit;
- Fig. 2a,b,c: eine schematische Darstellung der Komponenten einer erfindungsgemäßen tragbaren Gasanalysevorrichtung mit verschiedenen Ausführungsformen des Steuerelements;

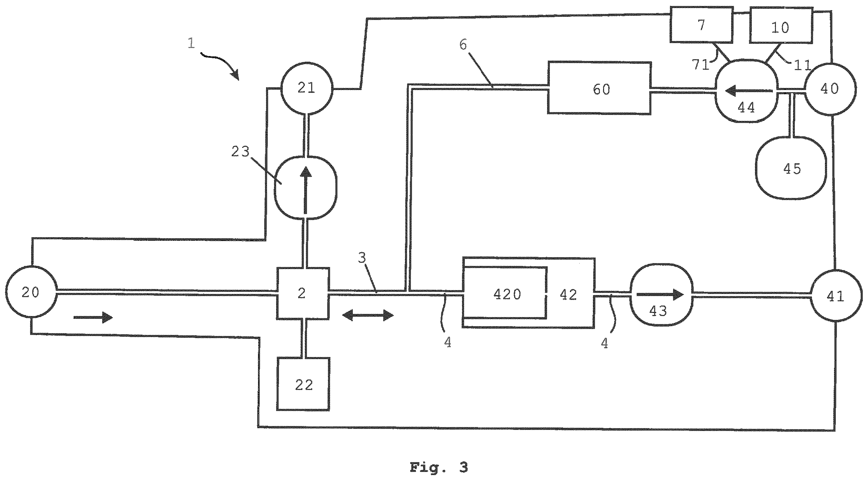

- Fig. 3: eine schematische Darstellung der Komponenten in einer alternativen Ausführungsform des Filterpfads;
- Fig. 4a,b,c: eine schematische Darstellung internen bzw. externen Auswerteeinheit und ihrer Verbindung mit den Detektoren und ggf. drahtlosen Verbindung mit Bedien- und Bildschirmelementen;
- Fig. 5a,b: eine schematische Darstellung der Komponenten und Gaswege in einer alternativen Ausführungsform mit Druckdifferenzsensor in zwei Zuständen abhängig von einer Ventilstellung; und
- Fig. 6: eine schematische Darstellung der Komponenten und Gaswege in Bezug auf Gaspfadverengungen, Druckdifferenzsensor sowie kombinierter Abluftöffnung.

Die tragbare Gasanalysevorrichtung wird in ihrer Gesamtheit mit dem Bezugszeichen 1 bezeichnet. Die Gasanalysevorrichtung 1 weist ein Gehäuse 13 auf, an dem eine Probengaseintrittsöffnung 20 und eine Trägergaseintrittsöffnung 40 angeordnet sind.

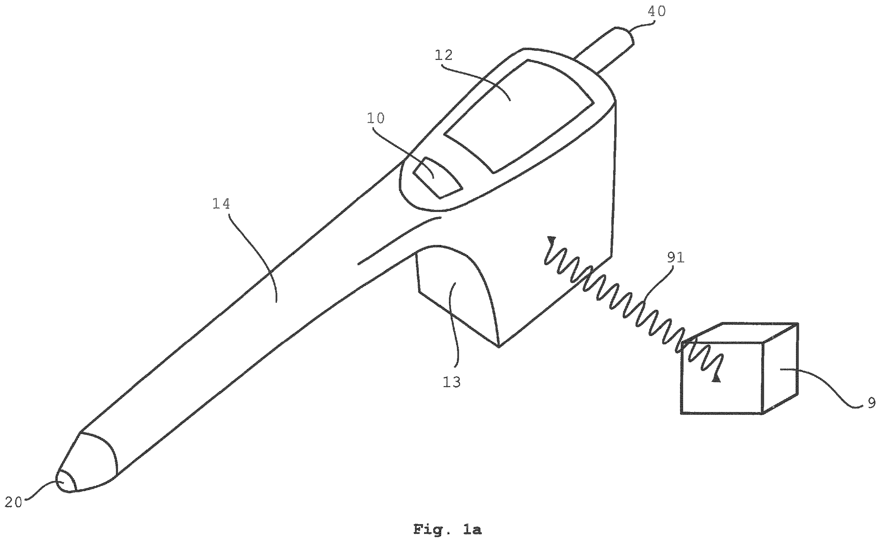

Bei einer ersten Ausführungsform, wie sie in Fig. 1a dargestellt ist, weist die Gasanalysevorrichtung ein kompaktes Gehäuse 13 auf. Weiter weist das kompakte Gehäuse 13 ein Bedienelement 10 sowie ein Bildschirmelement 12 auf und ist über eine drahtlose Verbindung 91 mit einer externen Auswerteeinheit 9 verbunden. Bedienelement 10 und Bildschirmelement 12 können vorzugsweise kombiniert ausgeführt sein, in Gestalt eines berührempfindlichen Displays ("Touch Screen"). Optional kann das Gehäuse 13, wie in Figur 1a dargestellt, pistolenförmig ausgeführt sein, wobei der Fortsatz 14 schaftförmig gestaltet ist. Notwendig ist dies aber nicht, das Gehäuse kann auch quaderartig ausgeführt sein, zugunsten einer noch größeren Kompaktheit.

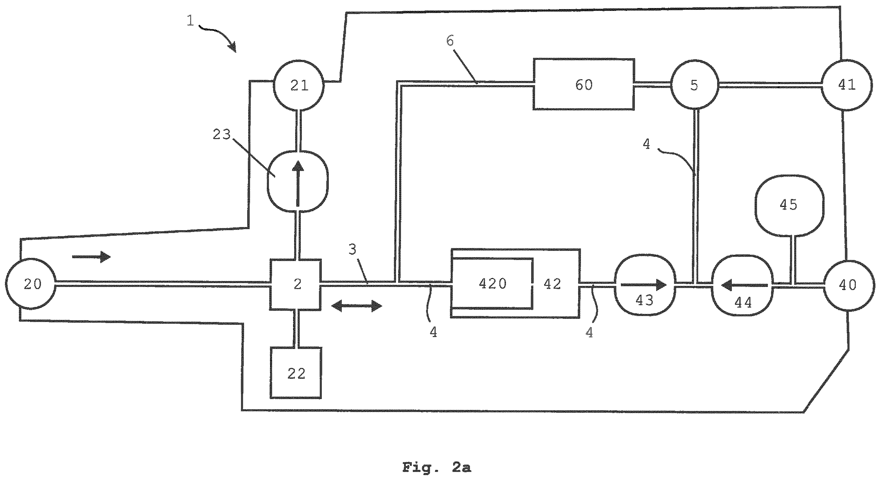

In einer weiteren Ausführungsform sind Bedienelement 10 und Bildschirmelement 12 extern angeordnet, vorzugsweise gemeinsam mit der Auswerteeinheit 9 in einem zweiten Gehäuse 92. In einer ersten bevorzugten Ausführungsform gemäß Figur 2a erstreckt sich von der Probengaseintrittsöffnung 20 ein Suchermesspfad 2 zu einer ersten Abluftöffnung 21. An der ersten Abluftöffnung 21 ist weiter eine erste regelbare Gebläseeinheit 23 als Flusserzeugerelement angeordnet. Die erste regelbare Gebläseeinheit 23 erzeugt einen Gasfluss in Richtung der ersten Abluftöffnung 21. Damit wird durch die erste regelbare Gebläseeinheit 23 Probengas von der Probengaseintrittsöffnung 20 angesaugt, das über den Suchermesspfad 2 zu der ersten Abluftöffnung 21 geleitet wird. Der Suchermesspfad 2 umfasst weiter einen Sucher-Detektor 22. Der Sucher-Detektor 22 ist ein Photoionisationsdetektor. Ein Teil des Probengasflusses wird auf den Sucher-Detektor 22 geleitet. Der Sucher-Detektor 22 führt dabei kontinuierlich eine Messung durch, mit der festgestellt werden kann, ob leicht flüchtige Verbindungen in dem Probengas enthalten sind.

Von dem Suchermesspfad 2 zweigt ein Verbindungspfad 3 ab. Von dem Verbindungspfad 3 erstreckt sich ein Trennmesspfad 4 bis zu einer zweiten Abluftöffnung 41. Im Trennmesspfad 4 ist ein Trenn-Detektor 42 angeordnet. Der Trenn-Detektor 42 ist ein Gaschromatograph, der als Trennsäule 420 eine Multi-Kapillar-Säule umfasst. Durch eine Multi-Kapillar-Säule kann ein großes Gasvolumen durch geleitet werden. Auf diese Weise können große Mengen an Probengas zum Trenn-Detektor 42 geleitet und retentiert werden. Die weiteren Komponenten der Gasanalysevorrichtung 1 bewirken keine Retention. Der aus Suchermesspfad 2, Verbindungspfad 3 und dem Abschnitt des Trennpfads zwischen Verbindungspfad 3 und Trenn-Detektor 42 gebildete Pfad wird als "analytischer Pfad" bezeichnet. Der analytische Pfad ist frei von Ventilen, d. h. er ist ventillos.

Nach ca. 30 Sekunden ist das gesamte Probengas durch den Trenn-Detektor 42 durchgeleitet und der Trennmesspfad 4 durch Trägergas gespült worden, so dass eine neue Messung der Einzel-Konzentrationen ohne Vorbelastung durch die vorherige Messung starten kann. Der Trenn-Detektor 42 ist weiter dazu ausgebildet, Benzolkonzentrationen mit einer Sensitivität von 25 ppb zu bestimmen.

Vom Verbindungspfad 3 aus gesehen, ist hinter dem Trenn-Detektor 42 eine zweite Gebläseeinheit 43 angeordnet. Die Gebläseeinheit 43 erzeugt einen Gasfluss vom Verbindungspfad 3 durch den Trenn-Detektor 42 in Richtung der zweiten Abluftöffnung 41.

Weiter ist der Trennmesspfad 4 mit der Trägergaseintrittsöffnung 40 verbunden, so dass Trägergas in den Trennmesspfad 4 geleitet werden kann. An der Trägergaseintrittsöffnung 40 ist eine dritte Gebläseeinheit 44 angeordnet, die einen Gasfluss von der Trägergaseintrittsöffnung 40 in Richtung des Trennmesspfads 4 erzeugt.

Von dem Verbindungspfad 3 zweigt ein Filterpfad 6 als Bypass zum Trenn-Detektor 42 ab. Der Bypass verbindet den Trennmesspfad 4 mit dem Verbindungsmesspfad 3. Der Trennmesspfad 4 und der Filterpfad 6 bilden dabei einen Kreislauf.

Der Filterpfad 6 umfasst weiter ein Filterelement 60. Das Filterelement 60 ist dazu ausgebildet, leicht flüchtige Verbindungen zu filtern. Wenn das Trägergas durch das Filterelement 60 fließt, werden alle leicht flüchtigen Verbindungen gefiltert, so dass das Trägergas nach Durchlaufen des Filterelements 60 keine leicht flüchtigen Verbindungen mehr enthält.

An der Verbindung zwischen dem Trennmesspfad 4 und dem Filterpfad 6 ist als Steuerelement ein Ventil 5 angeordnet. Das Ventil 5 ist vor der zweiten Abluftöffnung 41 angeordnet. Das Ventil 5 schaltet zwischen dem Filterpfad 6 und der zweiten Abluftöffnung 41. Das bedeutet, dass in einer Schaltstellung kein Gasfluss durch die zweite Abluftöffnung 41 ermöglicht wird. Der Gasfluss wird in diesem Fall von dem Trennmesspfad 4 in den Filterpfad 6 eingeleitet. In dieser Ausführungsform erzeugen die zweite und die dritte Gebläseeinheit 43, 44 insgesamt einen stärkeren Gasfluss als die erste Gebläseeinheit 23. Ein Teil des gefilterten Trägergases aus dem Filterpfad 6 wird dabei in den Trennmesspfad 4 geleitet und spült den Trenn-Detektor 42. Das Trägergas verbleibt dabei in dem durch den Trennmesspfad 4 und dem Filterpfad 6 gebildeten Kreislauf.

Der andere Teil des Gasflusses aus dem Filterpfad 6 wird durch den Verbindungsmesspfad 3 in den Suchermesspfad 2 geleitet. Die erste Gebläseeinheit 23 ist so eingestellt, dass sie den kombinierten Gasfluss aus dem Verbindungsmesspfad 3 sowie aus dem Suchermesspfad 2 aus der ersten Abluftöffnung 21 heraus leitet. Dadurch wird vermieden, dass das Trägergas durch die Probeneintrittsöffnung 20 herausgeblasen wird.

Wenn das Ventil 5 den Filterpfad 6 sperrt, wird der Kreislauf aus Trennmesspfad 4 und Filterpfad 6 unterbrochen. Weiter wird das Trägergas, das von der Trägergaseintrittsöffnung 40 einströmt, direkt zu der zweiten Abluftöffnung 41 geleitet. Es wird damit kein Gas von dem Filterpfad 6 in den Verbindungsmesspfad 3 geleitet. Dadurch strömt nun Probengas aus dem Suchermesspfad 2 durch den Verbindungsmesspfad 3. Die Strömungsrichtung des Gasflusses im Verbindungsmesspfad 3 wird dadurch umgekehrt.

Aus dem Verbindungsmesspfad 3 strömt das Probengas in den Trennmesspfad 4 und damit durch den Trenn-Detektor 42. Mittels des Trenn-Detektors 42 werden verschiedene Verbindungen im Probengasgemisch getrennt, so dass deren Konzentration separat erfasst werden kann. Da weiter Probengas im Suchermesspfad 2 von der Probengaseintrittsöffnung 20 zur ersten Abluftöffnung 21 strömt, wird der Sucher-Detektor 22 weiterhin mit Probengas versorgt. Eine Unterbrechung der Messung des Sucher-Detektors 22 durch das Bestimmen der Einzel-Konzentrationen der leicht flüchtigen Verbindungen im Trennmesspfad 4 wird damit vermieden.

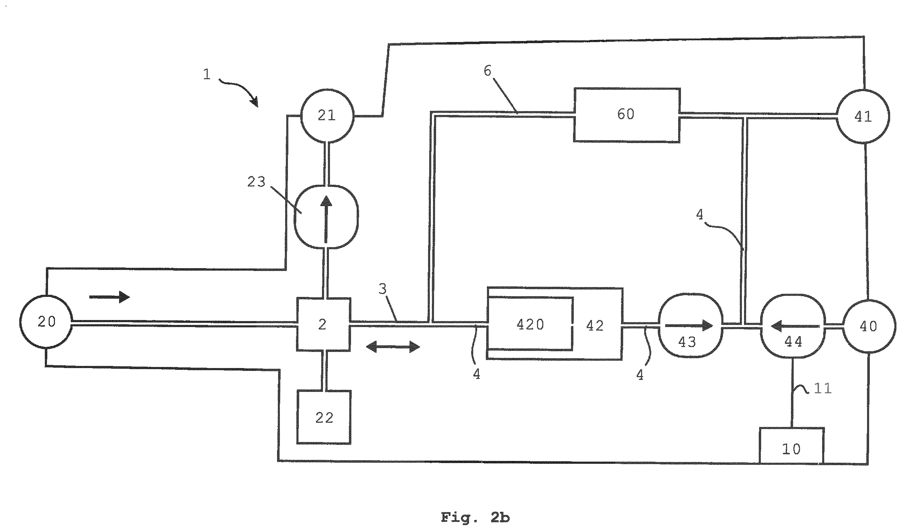

In einer zweiten bevorzugten Ausführungsform gemäß Figur 2b ist zwischen dem Filterpfad 6 und dem Suchermesspfad 4 kein Ventil vorgesehen. Stattdessen wird das Steuerelement durch die dritte regelbare Gebläseeinheit 44 gebildet. Die Richtung des Gasflusses im Verbindungsmesspfad 3 wird in dieser Ausführungsform durch die Gebläseleistung der dritten regelbaren Gebläseeinheit 44 gesteuert. Wenn die dritte regelbare Gebläseeinheit 44 bei hoher Leistung angesteuert wird, wird der Gasfluss durch die zweite Abluftöffnung 41 und in den Filterpfad 6 geleitet. Die Gebläseleistung der dritten regelbaren Gebläseeinheit 44 ist dabei so hoch, dass der Gasfluss aus dem Filterpfad 6 sowohl in den Verbindungsmesspfad 3 als auch in den Trennmesspfad 4 geleitet wird. Um den Gasfluss im Verbindungsmesspfad 3 umzukehren, wird die Leistung der dritten regelbaren Gebläseeinheit 44 so weit verringert, dass kein bzw. nur sehr wenig Trägergas in den Filterpfad 6 eingeleitet wird. Dadurch wird kein Trägergas von dem Filterpfad 6 in den Verbindungsmesspfad 3 eingeleitet. Stattdessen wird Probengas von dem Suchermesspfad 2 in den Verbindungsmesspfad 3 geleitet, so dass eine Umkehr des Gasflusses im Verbindungsmesspfad 3 stattfindet.

In einer alternativen Ausführungsform ist der Filterpfad 6 nicht als Bypass zum Trenn-Detektor 42 vorgesehen. Der Trennmesspfad 4 und der Filterpfad 6 bilden in dieser Ausführungsform keinen Kreislauf. Der Filterpfad 6 zweigt in dieser Ausführungsform von dem Verbindungspfad 3 ab und erstreckt sich bis zu der Trägergaseintrittsöffnung 40. Der Filterpfad 6 umfasst das Filterelement 60. An der Trägereintrittsöffnung 40 ist die dritte Gebläseeinheit 44 als Steuereinheit angeordnet. Die dritte Gebläseeinheit 44 leitet Trägergas von der Trägergaseintrittsöffnung 40 in Richtung des Verbindungspfads 3. Durch Auf- und Abregelung der dritten regelbaren Gebläseeinheit 44 kann die Richtung des Gasflusses im Verbindungspfad 3 gesteuert werden.

Anstatt der dritten regelbaren Gebläseeinheit 44 kann auch die erste regelbare Gebläseeinheit 23 oder die zweite regelbare Gebläseeinheit 43 als Steuerelement fungieren. Weiter kann auch eine Kombination der regelbaren Gebläseeinheiten 23, 43, 44 als Steuerelement genutzt werden. Die Gebläseeinheiten 23, 43, 44 fungieren als Flusserzeugerelemente.

In den Ausführungsformen, in denen das Steuerelement durch mindestens eine der regelbaren Gebläseeinheiten 23, 43, 44 gebildet wird, kann ein Durchflusssensor 45 an der Trägergaseintrittsöffnung 40 vorgesehen sein. Der Durchflusssensor 45 misst, die Menge des Trägergases, das durch die Trägergaseintrittsöffnung 40 in den Trennmesspfad 4 einströmt. Die regelbaren Gebläseeinheiten 23, 43, 44 können auf Basis der Durchflussmenge des Trägergases gesteuert werden, um eine Umkehr des Gasflusses im Verbindungspfad 3 zu erreichen.

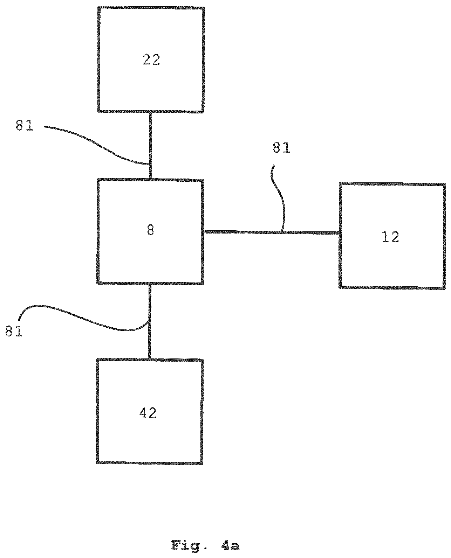

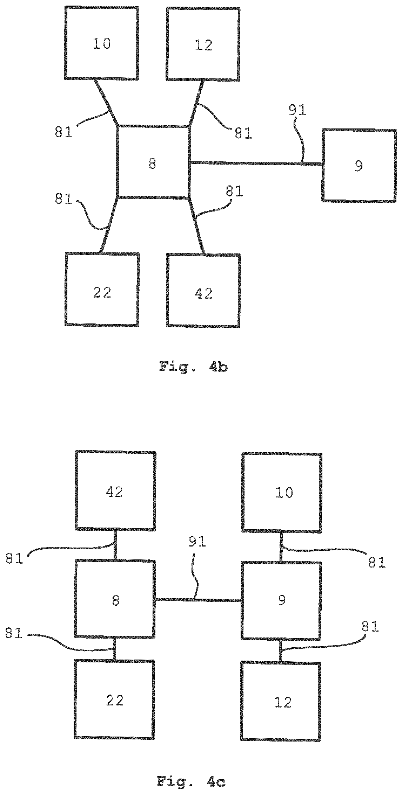

Weiter ist ein Ortungsmodul 7 vorgesehen, dass ausgebildet ist, die geographische Position der tragbaren Gasanalysevorrichtung 1 zu ermitteln. Das Ortungsmodul 7 ist über eine Ortungs-Signalleitung 71 mit dem Steuerelement verbunden. Über die Ortungs-Signalleitung 71 kann das Ortungsmodul 7 das Positionssignal an das Steuerelement übermitteln. Das Positionssignal kann dann in einer Speichereinheit (nicht dargestellt), die mit einer internen Auswerteeinheit 8 verbunden ist, mit den Messdaten der Detektoren 22, 42 gespeichert werden. Die Detektoren 22, 42 sind dabei über Datenleitungen 81 mit der internen Auswerteeinheit 8 verbunden (vgl. Figur 4). Auf diese Weise können die Messdaten der Detektoren 22, 42 mit den Positionsdaten verknüpft werden, so dass mit mehreren Messungen an verschiedenen geographischen Positionen eine räumliche Auflösung der Messdaten erfolgen kann.

Das Bedienelement 10 ist weiter über eine Auslösesignalleitung 11 mit dem Steuerelement verbunden. Über das Bedienelement 10 kann ein Benutzer manuell eine Umkehrung des Gasflusses im Verbindungspfad 3 anfordern. Auf diese Weise kann eine Messung der Einzel-Konzentrationen der leicht flüchtigen Verbindungen ausgelöst werden. Alternativ kann die Messung der Einzel-Konzentrationen automatisch ausgelöst werden, wenn der Sucher-Detektor 22 eine erhöhte Gesamt-Konzentration von leicht flüchtigen Verbindungen erfasst.

Die erfassten Messdaten können von der Auswerteeinheit 8 über eine Signalverbindung 81 an das Bildschirmelement 12 übermittelt werden. Das Bildschirmelement 12 kann diese Messdaten dem Benutzer anzeigen. Der Benutzer erhält damit vor Ort die Daten der Messung. Das Bildschirmelement 12 wird mit einem Zyklus von weniger als 1 Sekunde aktualisiert, d.h. es zeigt den aktuellen Verlauf der Messung durch den Trenn-Detektor 42 bzw. des Sucher-Detektors 22 an. Nach ca. 10 Sekunden Messdauer kann der Benutzer daher bereits entscheiden, ob ein auswertbarer Datensatz vorliegt oder nicht.

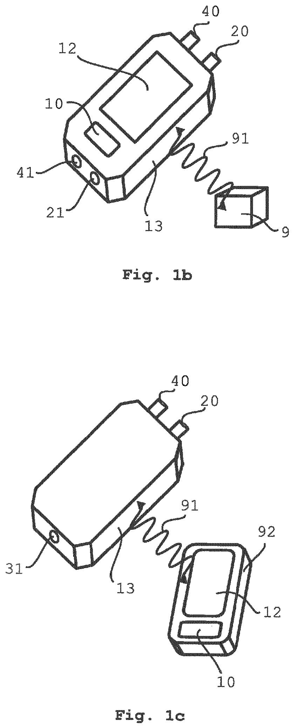

In einer weiteren Ausführungsform kann zusätzlich zu der internen Auswerteeinheit 8 eine externe Auswerteeinheit 9 vorgesehen werden. Die Messdaten der Detektoren 22, 42 werden über Datenverbindungen 81 an die interne Auswerteeinheit angelegt und weiter über eine drahtlose Verbindung 91 an die externe Auswerteeinheit 9 übermittelt. Bedienelement 10 sowie Bildschirmelement 12 sind dabei über Datenverbindungen 81 an die interne Auswerteeinheit 8 und/oder die externe Auswerteeinheit 9 verbunden. Beispielhafte Ausführungen von Gehäusen und Signalverbindungen sind in Fig. 1b in Verbindung mit Fig. 4b bzw. Fig. 1c in Verbindung mit Fig. 4c veranschaulicht.

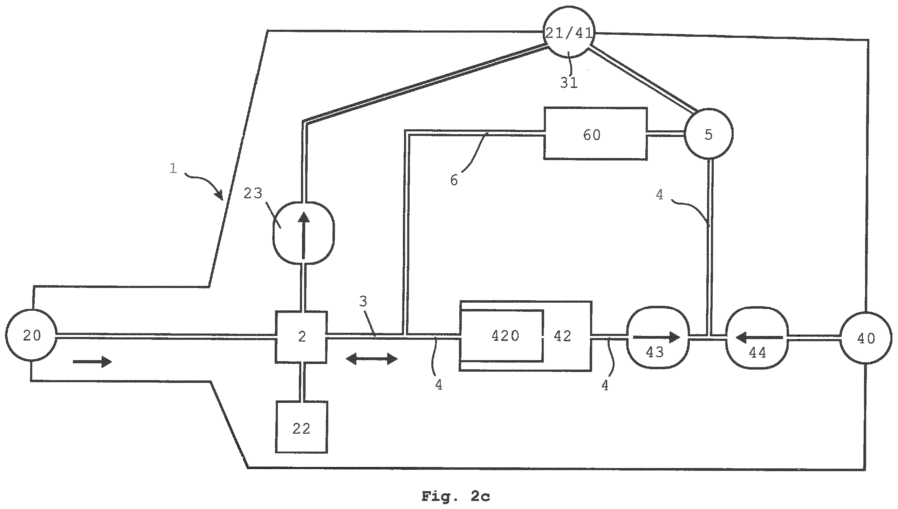

Es sei angemerkt, dass gewünschtenfalls die Abluftöffnungen 21, 41 kombiniert sein können zu einer gemeinsamen Abluftöffnung 31. Dies stellt eine weitere vorteilhafte Vereinfachung dar und ermöglicht eine sehr kompakte Ausführung, wie in Fig. 1c sowie Fig. 2c veranschaulicht.

Zur Bestimmung der Einzel-Konzentrationen der leicht flüchtigen Verbindungen wird der Gasfluss im Verbindungspfad 3 für ca. 1 Sekunde umgekehrt. Die auf diese Weise entnommene Menge an Probengas reicht für eine Messung durch den Trenn-Detektor 42 in der Regel aus. Auf Grund der kurzen Entnahmezeit kann ein Benutzer mit der Gasanalysevorrichtung 1 weitere Orte aufsuchen, an denen leicht flüchtige Verbindungen vermutet werden und diese mittels des Sucher-Detektors 22 detektieren.

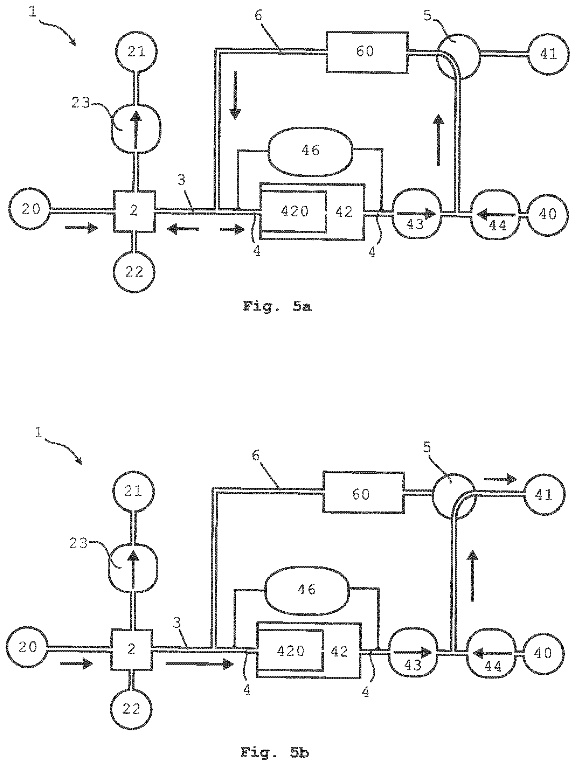

In einer alternativen Ausführungsform ist über dem Trenn-Detektor 42 ein Durchfluss- oder Druckdifferenzsensor 46 angeordnet, wobei ein Durchflusssensor direkt oder ein Druckdifferenzsensor indirekt den Gasfluss misst, der durch die regelbare Gebläseeinheit 43 erzeugt wird. Die Druckdifferenz ist unmittelbare Ursache des Gasflusses. Das Gebläse 43 wird von der internen Auswerteeinheit 8 in Abhängigkeit der Messung am Druckdifferenzsensor 46 geregelt. Eine Gebläseeinheit 23 erzeugt einen kontinuierlichen Gasfluss im Suchermesspfad 2 von der Probengaseintrittsöffnung 20 zur ersten Abluftöffnung 21. Für die Injektion der Trenn-Messung, deren Ventilstellung und Gasflüsse schematisch in Fig. 5b dargestellt sind, wird der Gasfluss im Verbindungsstück 3 von dem Suchermesspfad 2 in den Trennmesspfad 4 gerichtet und so Probengas aus der Probengaseintrittsöffnung 20 in die Trennsäule 420 des Trenn-Detektors 42 injiziert. In einer anderen Stellung am Ventil 5, die vor und nach der Injektion eingestellt wird, wird der Trennmesspfad 4 mit im Filter 60 gefiltertem Trägergas aus dem Filterpfad 6 gespült, wobei das Trägergas mit dem Gasfluss aus dem Trenn-Detektor zu einem stärkeren Gasfluss zusammengeführt werden kann, wie in Fig. 5a dargestellt. Vorzugsweise wird die Leistung der regelbaren Gebläseeinheit 43 in Abhängig der Messung am Durchfluss- oder Druckdifferenzsensor 46 so geregelt, dass eine vorgegebene Druckdifferenz und somit ein bestimmter Gasfluss durch die Trennsäule 420 erzeugt wird. Werden bei aufeinander folgenden Messungen jeweils gleiche Gasflüsse eingestellt, sind die Messungen besser vergleichbar.

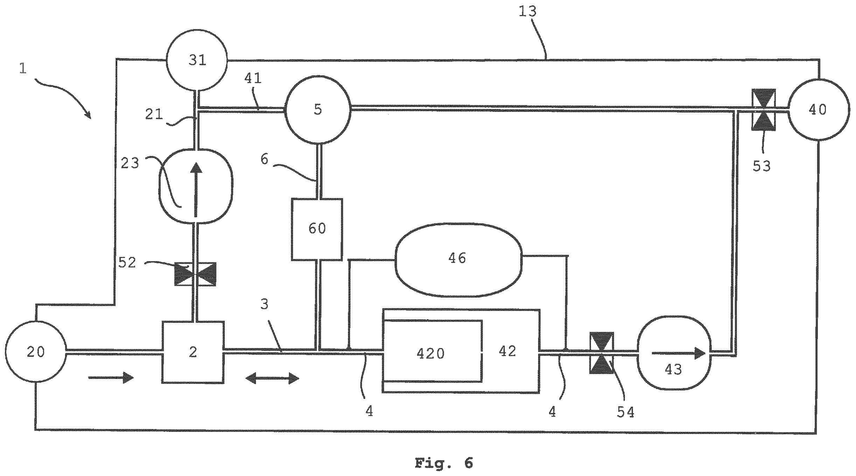

In einer alternativen Ausführungsform der Gasanalysevorrichtung, schematisch dargestellt in Fig. 6, ist der Trägergaszustrom passiv ausgeführt, also ohne eine dritte Gebläseeinheit 44. Außerdem ist der Suchermesspfad 2 dargestellt in einer Ausführung ohne Sucher-Detektor 22, die nicht unter die Ansprüche fällt. Der Entfall der dritten Gebläseeinheit 44 kann ggf. auch bei den übrigen Ausführungsformen vorgesehen sein. Damit ist eine aktive Komponente eingespart, wodurch Herstellungskosten, Energieverbrauch, Wärmeentwicklung und Ausfallrisiko reduziert werden. Der Gasfluss in den Pfaden 2, 3, 4, 6, 21, 41 wird von Druckdifferenzen im Gaswegesystem bestimmt, die von den beiden Gebläseeinheiten 23, 43 erzeugt werden. Verengungen der Gaspfade 52, 53, 54 sind so ausgebildet, dass passiv der Durchfluss verringert wird, wodurch die gewünschten Flussrichtungen in geeigneten Geschwindigkeiten und ohne Rückflüsse erreicht werden. Die Gaspfadverengungen 52, 53, 54 können durch geeignete Durchmesser der Schläuche oder Gasflussrestriktionen ausgeführt sein. Eine Gebläseeinheit 23 erzeugt einen kontinuierlichen Gasfluss im Suchermesspfad 2 von der Probengaseintrittsöffnung 20 über die erste Abluftöffnung 21 zur gemeinsamen Abluftöffnung 31. In einer ersten Stellung am Ventil 5 wird die Trägergaseintrittsöffnung 40 mit dem Filterpfad 6 und dem Trennmesspfad 4 verbunden. Die Leistungen der Gebläse 23, 43 und die Durchmesser der Gaspfadverengungen sind so gewählt, dass der Gasfluss im Verbindungspfad 3 vom Filterpfad 6 in den Suchermesspfad 2 stärker ist als der Gasfluss vom Filterpfad 6 in den Trennmesspfad 4. Das Volumen, das aus dem Kreislauf von Trennmesspfad 4 und Filterpfad 6 gebildet wird, hat in dieser Ventilstellung zwei Verbindungen, eine zum Verbindungspfad 3 und eine zur Trägergaseintrittsöffnung 40. Da der Gasfluss im Verbindungspfad 3 zum Suchermesspfad 2 gerichtet ist, muss an der Trägergaseintrittsöffnung 40 Trägergas angesaugt werden. Die Gaspfadverengung 53 verhindert zusätzlich eine turbulente Strömung an dieser Stelle, die einen kurzzeitigen Rückfluss verursachen könnte. Ein Durchfluss- oder Druckdifferenzsensor 46 kontrolliert den Zustand des Gasflusses im Trennmesspfad 4, wobei die Leistung der Gebläseeinheit 43 geregelt wird, um einen bestimmten Gasfluss zu erreichen. In einer zweiten Stellung am Ventil 5 werden Trägergaseintrittsöffnung 40 und Trennmesspfad 4 mit der zweiten Abluftöffnung 41 verbunden und die Verbindung zum Filterpfad 6 unterbrochen. Der Gasfluss von der Probengaseintrittsöffnung 20 ist stets in Richtung des Suchermesspfades 2 und von dort über zwei Pfade zur gemeinsamen Abluftöffnung 31 gerichtet. So wird sichergestellt, dass ein abgeschlossenes Probenvolumen nicht verunreinigt oder verdünnt wird und Gas nur über die Abluftöffnungen 21, 41 bzw. die gemeinsame Abluftöffnung 31 das Gerät verlässt. Der erste Pfad verläuft aus dem Suchermesspfad 2 über die Gebläseeinheit 23 und die erste Abluftöffnung 21. Der zweite Pfad verläuft über den Suchermesspfad 2 in den nun umgekehrt gerichteten Fluss im Verbindungspfad in den Trennmesspfad 4, wo Probengas in den Trenn-Detektor 42 injiziert wird. In dieser zweiten Ventilstellung wird auch der Gasfluss an der Trägergaseintrittsöffnung 40 umgerichtet, da dort ein Teil des Gasflusses aus dem Trennmesspfad 4 ausströmt. Vorzugsweise ist das Trägergas in dieser Ausführungsform Luft oder die Gaspfadverengung 53 ist so gewählt, dass das Trägergasvolumen außerhalb der Trägergaseintrittsöffnung 40 durch diesen Fluss aus dem Trennmesspfad 4 nicht übermäßig kontaminiert wird. Der Großteil der Abluft aus dem Trennmesspfad 4 strömt in die zweite Abluftöffnung 41. Die beiden Abluftöffnungen 21, 41 sind im Beispiel als gemeinsame Abluftöffnung 31 am Gehäuse 13 zusammengeführt. In dieser Ausführung werden auch mit wenigen aktiven Komponenten die Vorteile der erfindungsgemäßen Verbindung von Suchermesspfad 2 und Trennmesspfad 4 über den Verbindungspfad 3 erreicht, da diese Pfade alle ventillos ausgeführt sind und der kontinuierliche Betrieb des Gebläses 23 im Sucher und in der zweiten Ventilstellung die Gegenspülung mit gefiltertem Trägergas durch den Verbindungspfad 3 in den Suchermesspfad 2 jeweils eine Anreicherung der Gaspfade mit flüchtigen Verbindungen verhindern. Der kontinuierliche Betrieb der beiden Gebläseeinheiten 23, 43 stabilisiert deren Leistung verglichen mit einem Einschaltvorgang zu Beginn der Messung. Durch die Leistungsregelung des Gebläses 43 in Abhängigkeit der Messung des Druckdifferenzsensors 46 wird der Gasfluss weiter stabilisiert und nur durch die Stellung des Ventils 5 außerhalb des analytischen Pfads beeinflusst. Das Messgerät ist damit insgesamt in seinem Betrieb sehr stabil, wodurch aufeinanderfolgende Messungen und auch Signalwerte innerhalb einer Messung am Trenn-Detektor besser vergleichbar sind. In der zweiten Ventilstellung ist das Gerät in einem Bereitschaftsmodus, in dem stets ein Gasfluss von der Probengaseintrittsöffnung 20 in den Suchermesspfad 2 besteht. Nach dem Auslösen der Trenn-Messung am Bedienelement 10 wird ohne Verzögerung das Ventil 5 umgeschaltet und der Fluss im Verbindungspfad 3 umgekehrt, sodass Probengas direkt aus dem kontrollierten Gasfluss im Suchermesspfad 2 in den Trennmesspfad 4 injiziert wird und die Trenn-Messung beginnt. Zwischen Auslösen der Messung und Ende der Injektion vergeht etwa eine Sekunde. Ohne den Sucher müsste die Probe aus einem stehenden Volumen außerhalb des Gerätes injiziert werden, eine zeitaufwendigere und schlechter kontrollierbare Lösung, die nicht unter die Ansprüche fällt.

## Patentansprüche

1. Gasanalysevorrichtung zum Durchleiten eines Gasflusses, insbesondere für leicht flüchtige Verbindungen, wobei die Gasanalysevorrichtung einen Suchermesspfad (2) und einen Trennmesspfad (4) umfasst, wobei der Suchermesspfad (2) sich von einer Probeneintrittsöffnung (20) zu einer ersten Abluftöffnung (21) erstreckt, wobei ein Verbindungspfad (3) von dem Suchermesspfad (2) abzweigt zu dem Trennmesspfad (4), wobei der Trennmesspfad (4) sich vom Verbindungspfad (3) zu einer zweiten Abluftöffnung (41) erstreckt und mit einer Trägergaseintrittsöffnung (40) verbunden ist, und wobei am Trennmesspfad (4) ein Trenn-Detektor (42) angeordnet ist, **dadurch gekennzeichnet, dass** die Gasanalysevorrichtung (1) tragbar ist und ein Steuerelement (5, 23, 43, 44) aufweist, das zum Umkehren des Gasflusses im Verbindungspfad (3) derart ausgebildet ist, dass bei umgekehrter Richtung ein Teil von einem an der Probeneintrittsöffnung eintretenden Probengasgemisch über den Verbindungspfad (3) in den Trennmesspfad (4) fließt und der andere Teil dem Suchermesspfad (2) folgt, wobei im Suchermesspfad (2) ein Sucher-Detektor (22) angeordnet ist, der zum kontinuierlichen Erkennen der Anwesenheit einer Gruppe von leicht flüchtigen Verbindungen ausgebildet ist.

2. Tragbare Gasanalysevorrichtung nach Anspruch 1, **dadurch gekennzeichnet, dass** der Trenn-Detektor (42) zur Mengenbestimmung von leicht flüchtigen Verbindungen ausgebildet ist und vorzugsweise ein Gaschromatograph mit einem Photoionisationsdetektor, mit einem Wärmeleitfähigkeitsdetektor, mit einem Halbleiter-Gasdetektor oder mit einem Massenspektrometer ist.

3. Tragbare Gasanalysevorrichtung nach Anspruch 1 oder 2, **dadurch gekennzeichnet, dass** der Trenn-Detektor (42) eine Trennsäule (420) umfasst, wobei die Trennsäule (420) als Multi-Kapillar-Säule ausgebildet ist.

4. Tragbare Gasanalysevorrichtung nach einem der Ansprüche 1 bis 3, **dadurch gekennzeichnet, dass** der Suchermesspfad (2), der Trennmesspfad (4) bis zum Trenn-Detektor (42) und der Verbindungspfad (3) jeweils ventillos ausgeführt sind.

5. Tragbare Gasanalysevorrichtung nach einem der Ansprüche 1 bis 4, **dadurch gekennzeichnet, dass** der Sucher-Detektor (22) ein Photoionisationsdetektor, ein Halbleiter-Gasdetektor oder ein Wärmeleitfähigkeitsdetektor ist.

6. Tragbare Gasanalysevorrichtung nach einem der Ansprüche 1 bis 5, **dadurch gekennzeichnet, dass** die erste Abluftöffnung (21) und die zweite Abluftöffnung (41) innerhalb eines Gehäuses (13) kombiniert sind zu einer gemeinsamen Abluftöffnung (31).

7. Tragbare Gasanalysevorrichtung nach einem der Ansprüche 1 bis 6, **dadurch gekennzeichnet, dass** ein Filterpfad (6) vom Verbindungspfad (3) abzweigt, wobei in dem Filterpfad (6) ein Filterelement (60) angeordnet ist und wobei der Filterpfad (6) mit der Trägergaseintrittsöffnung (40) verbunden ist.

8. Tragbare Gasanalysevorrichtung nach Anspruch 7, **dadurch gekennzeichnet, dass** das Steuerelement (5) als ein Ventil ausgebildet ist, das zum Öffnen und Sperren des Filterpfads (6) ausgebildet ist.

9. Tragbare Gasanalysevorrichtung nach einem der Ansprüche 1 bis 7, **dadurch gekennzeichnet, dass** das Steuerelement (23, 43, 44) als eine regelbare Gebläseeinheit an der Trägergaseintrittsöffnung (40), an der ersten Abluftöffnung (21) und/oder an dem Trenn-Detektor (42) ausgebildet ist.

10. Tragbare Gasanalysevorrichtung nach einem der Ansprüche 1 bis 7, **dadurch gekennzeichnet, dass** an der ersten Abluftöffnung (21) eine erste regelbare Gebläseeinheit (23) als Flusserzeugerelement angeordnet ist, wobei die erste regelbare Gebläseeinheit (23) einen Gasfluss in Richtung der ersten Abluftöffnung (21) erzeugt, und zwischen dem Trenndetektor (42) und der zweiten Abluftöffnung (41) eine zweite Gebläseeinheit (43) angeordnet ist, die einen Gasfluss vom Verbindungspfad (3) durch den Trenndetektor (42) in Richtung der zweiten Abluftöffnung (41) erzeugt.

11. Tragbare Gasanalysevorrichtung nach einem der Ansprüche 1 bis 10, **dadurch gekennzeichnet, dass** über dem Trennmesspfad (4) ein Durchfluss- oder Druckdifferenzsensor (46) angeordnet ist, wobei die Leistung einer Gebläseeinheit am Trenn-Detektor (42) mittels einer Auswerteeinheit (8) in Abhängigkeit der Messung des Durchfluss- oder Druckdifferenzsensors (46) geregelt wird.

12. System, das eine tragbare Gasanalysevorrichtung (1) nach einem der Ansprüche 1 bis 11 mit einer internen Auswerteeinheit (8) und eine externe Auswerteeinheit (9) aufweist, **dadurch gekennzeichnet, dass** die interne Auswerteeinheit (8) der Gasanalysevorrichtung (1) und die externe Auswerteeinheit (9) zum Austausch von Steuerbefehlen und Messdaten mittels einer drahtlosen Signalverbindung ausgebildet sind.

13. Gasanalyseverfahren mittels einer tragbaren Gasanalysevorrichtung (1) nach einem der Ansprüche 1 bis 11 mit den Schritten:
Einstellen eines höheren Drucks im Trennmesspfad (4) relativ zum Suchermesspfad (2),
Erkennen der Anwesenheit einer leicht flüchtigen Verbindung aus einer Gruppe von leicht flüchtigen Verbindungen im Suchermesspfad (2) mittels des Sucher-Detektors(22),
Einstellen eines niedrigeren Drucks im Trennmesspfad (4) relativ zum Suchermesspfad (2) und Trennen der einzelnen Mitglieder der Gruppe von leicht flüchtigen Verbindungen.

14. Gasanalyseverfahren nach Anspruch 13, **dadurch gekennzeichnet, dass** das Erkennen der Anwesenheit der leicht flüchtigen Verbindungen während des Trennens und eines Bestimmens kontinuierlich weitergeführt wird.

## Claims

1. Gas analysis device for the passage of a gas flow, in particular for volatile compounds, the gas analysis device comprising a finder measurement path (2) and a separation measurement path (4), the finder measurement path (2) extending from a sample inlet opening (20) to a first exhaust air opening (21), a connecting path (3) branching off from the finder measurement path (2) to the separation measurement path (4), the separation measurement path (4) extending from the connecting path (3) to a second exhaust air opening (41) and being connected to a carrier gas inlet opening (40), and a separation detector (42) being arranged on the separation measurement path (4), **characterized in that** the gas analysis device (1) is portable and has a control element (5, 23, 43, 44) which is designed to reverse the gas flow in the connecting path (3) such that, in the reverse direction, a part of a sample gas mixture entering at the sample inlet opening flows via the connecting path (3) into the separation measurement path (4) and the other part follows the finder measurement path (2), a finder detector (22) being arranged in the finder measurement path (2), which finder detector is designed to continuously ascertain the presence of a group of volatile compounds.

2. Portable gas analysis device according to claim 1, **characterized in that** the separation detector (42) is designed to determine the quantity of volatile compounds and is preferably a gas chromatograph having a photoionization detector, a thermal conductivity detector, a semiconductor gas detector or a mass spectrometer.

3. Portable gas analysis device according to either claim 1 or claim 2, **characterized in that** the separation detector (42) comprises a separation column (420), the separation column (420) being designed as a multi-capillary column.

4. Portable gas analysis device according to any of claims 1 to 3, **characterized in that** the finder measurement path (2), the separation measurement path (4) up to the separation detector (42) and the connection path (3) are each designed without valves.

5. Portable gas analysis device according to any of claims 1 to 4, **characterized in that** the finder detector (22) is a photoionization detector, a semiconductor gas detector or a thermal conductivity detector.

6. Portable gas analysis device according to any of claims 1 to 5, **characterized in that** the first exhaust air opening (21) and the second exhaust air opening (41) are combined within a housing (13) to form a common exhaust air opening (31).

7. Portable gas analysis device according to any of claims 1 to 6, **characterized in that** a filter path (6) branches off from the connecting path (3), a filter element (60) being arranged in the filter path (6) and the filter path (6) being connected to the carrier gas inlet opening (40).

8. Portable gas analysis device according to claim 7, **characterized in that** the control element (5) is designed as a valve, which is designed to open and close the filter path (6).

9. Portable gas analysis device according to any of claims 1 to 7, **characterized in that** the control element (23, 43, 44) is designed as a controllable blower unit at the carrier gas inlet opening (40), at the first exhaust air opening (21) and/or at the separation detector (42).

10. Portable gas analysis device according to any of claims 1 to 7, **characterized in that** a first controllable blower unit (23) is arranged as a flow generating element at the first exhaust air opening (21), the first controllable blower unit (23) generating a gas flow in the direction of the first exhaust air opening (21), and a second blower unit (43) being arranged between the separation detector (42) and the second exhaust air opening (41), which second blower unit generates a gas flow from the connection path (3) through the separation detector (42) in the direction of the second exhaust air opening (41).

11. Portable gas analysis device according to any of claims 1 to 10, **characterized in that** a flow or pressure difference sensor (46) is arranged above the separation measurement path (4), the power of a blower unit at the separation detector (42) being controlled in a closed-loop manner by means of an evaluation unit (8) on the basis of the measurement of the flow or pressure difference sensor (46).

12. System comprising a portable gas analysis device (1) according to any of claims 1 to 11 having an internal evaluation unit (8) and an external evaluation unit (9), **characterized in that** the internal evaluation unit (8) of the gas analysis device (1) and the external evaluation unit (9) are designed to exchange control commands and measurement data by means of a wireless signal connection.

13. Gas analysis method using a portable gas analysis device (1) according to any of claims 1 to 11, comprising the steps of:
setting a higher pressure in the separation measurement path (4) relative to the finder measurement path (2),
ascertaining the presence of a volatile compound from a group of volatile compounds in the finder measurement path (2) by means of the finder detector (22), setting a lower pressure in the separation measurement path (4) relative to the finder measurement path (2) and separating the individual members of the group of volatile compounds.

14. Gas analysis method according to claim 13, **characterized in that** ascertaining the presence of the volatile compounds is carried out continuously during the separation and a determination.

## Revendications

1. Dispositif d'analyse de gaz pour le passage d'un flux de gaz, en particulier pour des composés hautement volatils, dans lequel le dispositif d'analyse de gaz comprend un chemin de mesure de recherche (2) et un chemin de mesure de séparation (4), dans lequel le chemin de mesure de recherche (2) s'étend depuis une ouverture d'entrée d'échantillon (20) jusqu'à une première ouverture d'évacuation d'air (21), dans lequel un chemin de liaison (3) bifurque à partir du chemin de mesure de recherche (2) jusqu'au chemin de mesure de séparation (4), dans lequel le chemin de mesure de séparation (4) s'étend depuis le chemin de liaison (3) jusqu'à une seconde ouverture d'évacuation d'air (41) et est relié à une ouverture d'entrée de gaz porteur (40), et dans lequel un détecteur de séparation (42) est disposé sur le chemin de mesure de séparation (4), **caractérisé en ce que** le dispositif d'analyse de gaz (1) est portable et présente un élément de commande (5, 23, 43, 44) qui est conçu pour inverser le flux de gaz dans le chemin de liaison (3) de telle sorte que, dans le sens inverse, une partie d'un mélange de gaz d'échantillon entrant au niveau de l'ouverture d'entrée d'échantillon s'écoule dans le chemin de mesure de séparation (4) par l'intermédiaire du chemin de liaison (3) et l'autre partie suit le chemin de mesure de recherche (2), dans lequel un détecteur de recherche (22) est disposé dans le chemin de mesure de recherche (2), lequel détecteur de recherche est configuré pour détecter en continu la présence d'un groupe de composés hautement volatils.

2. Dispositif d'analyse de gaz portable selon la revendication 1,
**caractérisé en ce que** le détecteur de séparation (42) est configuré pour déterminer la quantité de composés hautement volatils et est de préférence un chromatographe en phase gazeuse comportant un détecteur à photo-ionisation, comportant un détecteur de conductivité thermique, comportant un détecteur de gaz à semi-conducteur ou comportant un spectromètre de masse.

3. Dispositif d'analyse de gaz portable selon la revendication 1 ou 2, **caractérisé en ce que** le détecteur de séparation (42) comprend une colonne de séparation (420), dans lequel la colonne de séparation (420) est conçue comme une colonne multicapillaire.

4. Dispositif d'analyse de gaz portable selon l'une des revendications 1 à 3, **caractérisé en ce que** le chemin de mesure de recherche (2), le chemin de mesure de séparation (4) jusqu'au détecteur de séparation (42) et le chemin de liaison (3) sont respectivement réalisés sans vanne.

5. Dispositif d'analyse de gaz portable selon l'une des revendications 1 à 4, **caractérisé en ce que** le détecteur de recherche (22) est un détecteur à photo-ionisation, un détecteur de gaz à semi-conducteur ou un détecteur de conductivité thermique.

6. Dispositif d'analyse de gaz portable selon l'une des revendications 1 à 5, **caractérisé en ce que** la première ouverture d'évacuation d'air (21) et la seconde ouverture d'évacuation d'air (41) sont combinées à l'intérieur d'un boîtier (13) pour former une ouverture d'évacuation d'air commune (31).

7. Dispositif d'analyse de gaz portable selon l'une des revendications 1 à 6, **caractérisé en ce qu'**un chemin de filtrage (6) bifurque depuis le chemin de liaison (3), dans lequel un élément filtrant (60) est disposé dans le chemin de filtrage (6) et dans lequel le chemin de filtrage (6) est relié à l'ouverture d'entrée de gaz porteur (40).

8. Dispositif d'analyse de gaz portable selon la revendication 7,
**caractérisé en ce que** l'élément de commande (5) est conçu en tant que vanne conçue pour ouvrir et fermer le chemin de filtrage (6).

9. Dispositif d'analyse de gaz portable selon l'une des revendications 1 à 7, **caractérisé en ce que** l'élément de commande (23, 43, 44) est conçu en tant qu'unité formant soufflante réglable au niveau de l'ouverture d'entrée de gaz porteur (40), au niveau de la première ouverture d'évacuation d'air (21) et/ou au niveau du détecteur de séparation (42).

10. Dispositif d'analyse de gaz portable selon l'une des revendications 1 à 7, **caractérisé en ce qu'**une première unité formant soufflante réglable (23) est disposée au niveau de la première ouverture d'évacuation d'air (21) en tant qu'élément générateur de flux, dans lequel la première unité formant soufflante réglable (23) génère un flux de gaz en direction de la première ouverture d'évacuation d'air (21), et une seconde unité formant soufflante (43) est disposée entre le détecteur de séparation (42) et la seconde ouverture d'évacuation d'air (41), laquelle seconde unité formant soufflante génère un flux de gaz depuis le chemin de liaison (3) à travers le détecteur de séparation (42) en direction de la seconde ouverture d'évacuation d'air (41).

11. Dispositif d'analyse de gaz portable selon l'une des revendications 1 à 10, **caractérisé en ce qu'**un capteur de débit ou de différence de pression (46) est disposé au-dessus du chemin de mesure de séparation (4), dans lequel la puissance d'une unité formant soufflante au niveau du détecteur de séparation (42) est réglée au moyen d'une unité d'évaluation (8) en fonction de la mesure du capteur de débit ou de différence de pression (46).

12. Système qui présente un dispositif d'analyse de gaz (1) portable selon l'une des revendications 1 à 11 comportant une unité d'évaluation interne (8) et une unité d'évaluation externe (9), **caractérisé en ce que** l'unité d'évaluation interne (8) du dispositif d'analyse de gaz (1) et l'unité d'évaluation externe (9) sont configurées pour échanger des instructions de commande et des données de mesure au moyen d'une connexion par signaux sans fil.

13. Procédé d'analyse de gaz au moyen d'un dispositif d'analyse de gaz (1) portable selon l'une des revendications 1 à 11, comportant les étapes consistant à :
régler une pression plus élevée dans le chemin de mesure de séparation (4) par rapport au chemin de mesure de recherche (2),
détecter la présence d'un composé hautement volatil d'un groupe de composés hautement volatils dans le chemin de mesure de recherche (2) au moyen du détecteur de recherche (22), régler une pression plus faible dans le chemin de mesure de séparation (4) par rapport au chemin de mesure de recherche (2) et séparer les membres individuels du groupe de composés hautement volatils.

14. Procédé d'analyse de gaz selon la revendication 13,
**caractérisé en ce que** la détection de la présence des composés hautement volatils est poursuivie en continu pendant la séparation et une détermination.
